(19) Europäisches Patentamt
European Patent Office
Office européen des brevets

(11) Veröffentlichungsnummer: **0 223 141 A1**

(12) # EUROPÄISCHE PATENTANMELDUNG

(21) Anmeldenummer: 86115193.4

(22) Anmeldetag: 03.11.86

(51) Int. Cl.[4]: C07D 277/82 , C07D 263/58 , A01N 43/78 , A01N 43/76

(30) Priorität: 13.11.85 JP 252823/85

(43) Veröffentlichungstag der Anmeldung: 27.05.87 Patentblatt 87/22

(84) Benannte Vertragsstaaten: BE CH DE FR GB IT LI NL

(71) Anmelder: NIHON TOKUSHU NOYAKU SEIZO K.K.
No.4, 2-chome, Nihonbashi Honcho Chuo-ku
Tokyo 103(JP)

(72) Erfinder: Kume, Toyohiko
6-7-8, Asahigaoka
Hino-shi Tokyo(JP)
Erfinder: Tsuboi, Shinichi
3-26-1, Hirayama
Hino-shi Tokyo(JP)
Erfinder: Sasaki, Shoko
1-7-3-1341, Higashi-Hirayama
Hino-shi§Tokyo(JP)
Erfinder: Hattori, Yumi
598, Kobiki-cho
Hachioji-shi Tokyo(JP)
Erfinder: Yagi, Shigeki
1-30-7, Izumi
Suginami-ku Tokyo(JP)

(74) Vertreter: Schumacher, Günter, Dr. et al
c/o Bayer AG Konzernverwaltung RP Patentabteilung
D-5090 Leverkusen 1 Bayerwerk(DE)

(54) **Neue Benzimidate.**

(57) Offenbart werden neue Benzimidate der Formel (I)

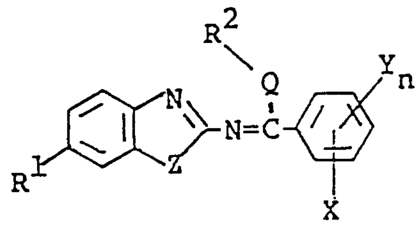

(I)

und die Verwendung der neuen Verbindungen als Insektizide oder Akarizide.

EP 0 223 141 A1

# Neue Benzimidate

Die vorliegende Erfindung betrifft neue Benzimidate, Verfahren zu ihrer Herstellung sowie ihre Verwendung als Insektizide und Akarizide.

Es wurde bereits offenbart, daß ein bestimmtes Benzamid insektizide Aktivitäten besitzt (siehe J. Agr. Food Chem. 24, 1976, 1065-1068).

Nunmehr wurden neue Benzimidate der Formel (I)

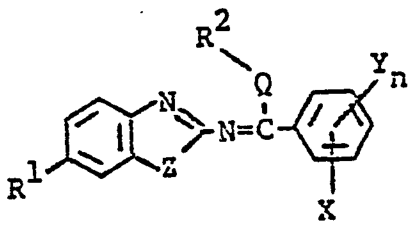

(I)

gefunden, in der

$R^1$ Halogen, Halogenoalkyl, Halogenoalkoxy, Halogenoalkylthio, Halogenoalkylsulfinyl oder Halogenoalkylsulfonyl bezeichnet,

X Halogen oder Alkyl bezeichnet,

Z Sauerstoff oder Schwefel bezeichnet,

Y Halogen oder Alkyl bezeichnet,

n 0, 1 oder 2 ist,

Q Sauerstoff, Schwefel oder die Gruppe >N-$R^3$ bezeichnet, in der

$R^3$ Wasserstoff oder Alkyl bezeichnet, und

$R^2$ Wasserstoff, Alkyl, Halogenoalkyl, Alkoxyalkyl, Alkylthioalkyl, N,N-disubstituiertes Aminoalkyl, Aralkyl, Aryloxyalkyl, Arylthioalkyl, Cycloalkyl, Alkenyl, Alkinyl, Aryl, Alkylthio, Aralkylthio, Arylthio, Alkylcarbonyl, Alkoxycarbonyl, Aryloxycarbonyl oder Cyano bezeichnet.

Die Verbindungen der Formel (I) werden mittels eines Verfahrens erhalten, bei dem

(a) in dem Fall, in dem $R^2$ in der Formel (I) Wasserstoff, Alkyl, Alkenyl, Alkinyl, Aryl, Cycloalkyl, Alkoxyalkyl, Alkylthioalkyl, Aryloxyalkyl, Arylthioalkyl, Aralkyl, Halogenoalkyl oder N,N-disubstituiertes Aminoalkyl bezeichnet, wobei in diesem Fall in der nachstehenden Formel (III) $R^2$ durch das Symbol $R^4$ ersetzt ist,

Verbindungen der Formel (II)

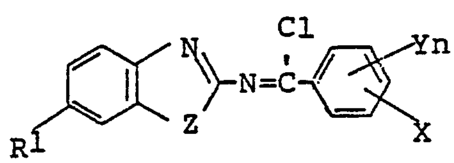

(II)

in der

$R^1$, X, Y, Z und n die gleichen Bedeutungen wie oben angegeben haben,

mit den Verbindungen der Formel (III)

$R^4$-Q-H (III),

in der $R^4$ und Q die im Vorstehenden angegebenen Bedeutungen haben, in Gegenwart inerter Lösungsmittel und in Gegenwart einer Base umgesetzt werden oder

(b) in dem Fall, in dem $R^2$ in der Formel (I) Alkyl, Alkenyl, Alkinyl, Cycloalkyl, Alkoxyalkyl, Alkylthioalkyl, Aryloxyalkyl, Arylthioalkyl, Aralkyl, Halogenoalkyl oder N,N-disubstituiertes Aminoalkyl bezeichnet und Q in der Formel (I) Schwefel ist, wobei in diesem Fall in der nachstehenden Formel (V) $R^2$ durch das Symbol $R^5$ ersetzt ist,

Verbindungen der Formel (IV)

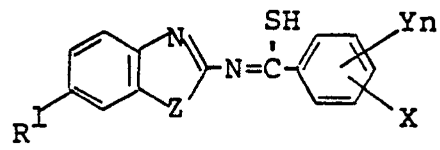 (IV) ,

in der

R', X, Y, Z und n die gleichen Bedeutungen wie oben angegeben haben,
mit Verbindungen der Formel (V)

$R^5$-M (V),

in der $R^5$ die im Vorstehenden angegebenen Bedeutungen hat und M Brom, Iod oder $-OSO_2-L$ bezeichnet, worin L für Alkyl oder Aryl steht, in Gegenwart inerter Lösungsmittel, gegebenenfalls in Gegenwart einer Base, umgesetzt werden oder

(c) in dem Fall, in dem $R^2$ in der Formel (I) Alkylthio, Arylthio, Alkylcarbonyl, Arylalkylthio, Alkoxycarbonyl oder Aryloxycarbonyl bezeichnet und Q in der Formel (I) Schwefel ist, wobei in diesem Fall in der nachstehenden Formel (VI) $R^2$ durch $R^6$ ersetzt ist, die vorstehenden Verbindungen der Formel (IV) mit Verbindungen der Formel (VI)

$R^6$-Cl (VI),

in der $R^6$ die im Vorstehenden angegebenen Bedeutungen hat; in Gegenwart inerter Lösungsmittel und in Gegenwart einer Base umgesetzt werden oder

(d) in dem Fall, in dem in der Formel (I) $R^2$ Cyano ist und Q Schwefel ist, die vorstehenden Verbindungen der Formel (II) mit Thiocyanaten in Gegenwart inerter Lösungsmittel umgesetzt werden oder

(e) in dem Fall, in dem in der Formel (I) $R^2$ Cyano ist und Q Schwefel ist, Verbindungen der Formel - (VII)

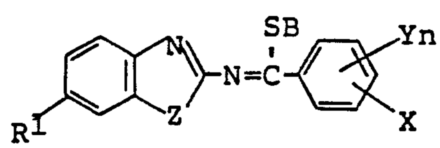 (VII) ,

in der

R', X, Y, Z und n die gleichen Bedeutungen wie oben angegeben haben und B ein Alkalimetall, ein Äquivalent eines Erdalkalimetalls oder eine Ammonium-Gruppe bezeichnet, mit Bromocyan in Gegenwart inerter Lösungsmittel umgesetzt werden oder

(f) in dem Fall, in dem $R^2$ in der Formel (I) die im Vorstehenden in der Verfahrensvariante (a) für $R^4$ angegebenen Bedeutungen hat und Q für $>N-R^3$ steht, Verbindungen der Formel (Ib)

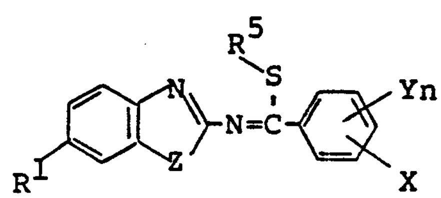 (Ib) ,

in der

R', X, Y, Z und n die gleichen Bedeutungen wie oben angegeben haben und $R^5$ die im Vorstehenden in der Verfahrensvariante (b) angegebenen Bedeutungen hat, mit Verbindungen der Formel (VIII)

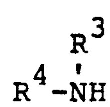 (VIII) ,

in der

$R^3$ und $R^4$ die im Vorstehenden angegebenen Bedeutungen haben, in Gegenwart inerter Lösungsmittel umgesetzt werden.

Die neuen Benzimidate zeigen potente insektizide Eigenschaften und auch akarizide Eigenschaften.

Überraschenderweise zeigen die erfindungsgemäßen Benzimidate eine wesentlich stärkere insektizide Wirkung als diejenigen, die aus dem oben genannten Stand der Technik bekannt sind, und insbesondere zeigen die Benzimidate außerordentlich überlegene Aktivitäten gegen Larven von Lepidoptera.

In der allgemeinen Formel sind die Alkyl-, Halogenoalkyl-, Alkylthio-, Halogenoalkoxy-, Halogenoalkylthio-, Halogenoalkylsulfinyl-und Halogenoalkylsulfonyl-Gruppen geradkettig oder verzweigt und enthalten vorzugsweise 1 bis 8, insbesondere 1 bis 6 und besonders bevorzugt 1 bis 4 Kohlenstoff-Atome.

Methyl, Ethyl, n-und i-Propyl sowie n-, sec-, i-und t-Butyl seien als Beispiele für die Alkyl-Gruppen oder die Alkyl-Teile der anderen Gruppen genannt. Halogenoalkyl-, Halogenoalkoxy-, Halogenoalkylthio-, Halogenoalkylsulfinyl-und Halogenoalkylsulfonyl-Gruppen enthalten vorzugsweise 1 bis 5, insbesondere 1 bis 3 gleiche oder verschiedene Halogen-Atome (vorzugsweise Fluor, Chlor oder Brom, insbesondere Fluor oder Chlor und besonders bevorzugt Fluor).

In der allgemeinen Formel enthalten die Alkoxyalkyl-, Alkylthioalkyl-, Alkylcarbonyl-und Alkoxycarbonyl-Gruppen, die geradkettig oder verzweigt sind, vorzugsweise 2 bis 8, insbesondere 2 bis 6 und besonders bevorzugt 2 bis 5 Kohlenstoff-Atome. Methoxyethyl, Ethoxyethyl, Methylthiomethyl, Ethylthioethyl, Methylcarbonyl, Ethylcarbonyl, Methoxycarbonyl und Ethoxycarbonyl seien als Beispiele erwähnt.

In den N,N-disubstituierten Aminoalkyl-Gruppen haben die Alkyl-Gruppen die gleichen bevorzugten Bedeutungen, wie sie oben für die Alkyl-Gruppen angegeben sind. Bevorzugte Substituenten (die gleich oder verschieden sein können) sind Alkyl-Gruppen mit vorzugsweise 1 bis 4, insbesondere 1 oder 2, Kohlenstoff-Atomen. Das Amino-Stickstoff-Atom kann auch durch eine Alkylen-Gruppe mit vorzugsweise 4 bis 6, insbesondere 4 oder 5, Kohlenstoff-Atomen substituiert sein (und auf diese Weise Teil eines 5-bis 7-, insbesondere 5-oder 6-, -gliedrigen Ringes sein). Dimethylaminoethyl und Pyrrolidinethyl seien als Beispiele erwähnt.

Die Cycloalkyl-Gruppen enthalten vorzugsweise 3 bis 7, insbesondere 3 bis 6 und besonders bevorzugt 5 oder 6 Kohlenstoff-Atome. Cyclopropyl, Cyclobutyl, Cyclopentyl und Cyclohexyl seien als Beispiele erwähnt.

Die Alkenyl-und Alkinyl-Gruppen sind geradkettig oder verzweigt und enthalten vorzugsweise 2 bis 6, insbesondere 2 bis 3, Kohlenstoff-Atome. Allyl und Propargyl seien als Beispiele erwähnt.

Aryl als Aryl-Gruppe und in dem Aryl-Teil von Aralkyl, Aryloxyalkyl, Arylthioalkyl, Arylalkylthio, Arylthio und Aryloxycarbonyl bezeichnet vorzugsweise Phenyl, das substituiert oder unsubstituiert (insbesondere unsubstituiert) sein kann. Die Alkyl-Teile der betreffenden Reste sind geradkettig oder verzweigt und enthalten vorzugsweise 1 bis 3, insbesondere 1 oder 2 Kohlenstoff-Atome. Das substituierte Phenyl enthält vorzugsweise 1 bis 3, insbesondere 1 oder 2 gleiche oder verschiedene Substituenten. Bevorzugte Substituenten sind Halogen (Fluor, Chlor und Brom, vorzugsweise Fluor oder Chlor), Alkyl, Alkoxy oder Alkylthio mit 1 bis 4, insbesondere 1 oder 2 Kohlenstoff-Atomen, die durch 1 bis 5, vorzugsweise 1 bis 3, Halogen-Atome (vorzugsweise Fluor oder Chlor) substituiert sein können, Nitro oder Cyano.

Sofern nichts anderes angegeben ist, bezeichnet Halogen Fluor, Chlor, Brom oder Iod, vorzugsweise Fluor, Chlor oder Brom, insbesondere Fluor oder Chlor.

In den allgemeinen Formeln bezeichnet Z vorzugsweise Schwefel.

In den allgemeinen Formeln bezeichnet $R^1$ vorzugsweise Fluor, Chlor, $C_1$-$C_2$-Fluoroalkyl, $C_1$-$C_2$-Fluoroalkoxy, $C_1$-$C_2$-Fluoroalkylthio, $C_1$-$C_2$-Fluoroalkylsulfinyl oder $C_1$-$C_2$-Fluoroalkylsulfonyl.

In den allgemeinen Formeln bezeichnet X vorzugsweise Fluor, Chlor oder Methyl.

In den allgemeinen Formeln bezeichnet Z vorzugsweise ein Schwefel-Atom.

In den allgemeinen Formeln bezeichnet Y vorzugsweise Fluor, Chlor oder Methyl.

In den allgemeinen Formeln bezeichnet Q vorzugsweise ein Sauerstoff-oder Schwefel-Atom oder die Gruppe $\geq$N-$R^3$, in der $R^3$ ein Wasserstoff-Atom oder $C_1$-$C_4$ Alkyl ist.

In den allgemeinen Formeln bezeichnet $R^2$ vorzugsweise Wasserstoff, $C_1$-$C_4$-Alkyl, $C_2$-$C_3$-Alkenyl, $C_2$-$C_3$-Alkinyl, Phenyl, $C_5$-$C_6$-Cycloalkyl, Alkoxyalkyl mit insgesamt 2 bis 4 Kohlenstoff-Atomen, Alkylthioalkyl mit insgesamt 2 bis 4 Kohlenstoff-Atomen, Phenoxy-$C_1$-$C_2$-alkyl, Phenylthio-$C_1$-$C_2$-alkyl, Benzyl, $C_1$-$C_2$-Fluoroalkyl oder -chloroalkyl, N,N-Dimethylaminoethyl, Pyrrolidinylethyl, $C_1$-$C_4$-Alkylthio, Phenylthio, Methylphenylthio, Chlorophenylthio, Alkylcarbonyl mit 1 bis 4 Kohlenstoff-Atomen in der Alkyl-Struktureinheit, Phenylmethylthio, Cyano, Alkylcarbonyl mit 1 bis 4 Kohlenstoff-Atomen in der Alkyl-Struktureinheit oder Phenoxycarbonyl.

Unter den erfindungsgemäßen Benzimidaten der Formel (I) sind bevorzugte Verbindungen diejenigen, in denen

$R^1$ Fluor, Chlor, $C_1$-$C_2$-Fluoroalkyl, $C_1$-$C_2$-Fluoroalkoxy, $C_1$-$C_2$-Fluoroalkylthio, $C_1$-$C_2$-Fluoroalkylsulfinyl oder $C_1$-$C_2$-Fluoroalkylsulfonyl bezeichnet,

X Fluor, Chlor oder Methyl bezeichnet,
Z ein Schwefel-Atom bezeichnet,
Y Fluor, Chlor oder Methyl bezeichnet,
n 0, 1 oder 2 ist,
Q ein Sauerstoff-oder Schwefel-Atom oder die Gruppe >N-$R^3$, in der $R^3$ ein Wasserstoff-Atom oder $C_1$-$C_4$-Alkyl ist, bezeichnet und
$R^2$ Wasserstoff, $C_1$-$C_4$-Alkyl, $C_2$-$C_3$-Alkenyl, $C_2$-$C_3$-Alkinyl, Phenyl, $C_5$-$C_6$-Cycloalkyl, Alkoxyalkyl mit insgesamt 2 bis 4 Kohlenstoff-Atomen, Alkylthioalkyl mit insgesamt 2 bis 4 Kohlenstoff-Atomen, Phenoxy-$C_1$-$C_2$-alkyl, Phenylthio-$C_1$-$C_2$-alkyl, Benzyl, $C_1$-$C_2$-Fluoroalkyl oder -chloroalkyl, N,N-Dimethylaminoethyl, Pyrrolidinylethyl, $C_1$-$C_4$-Alkylthio, Phenylthio, Methylphenylthio, Chlorophenylthio, Alkylcarbonyl mit 1 bis 4 Kohlenstoff-Atomen in der Alkyl-Struktureinheit, Phenylmethylthio, Cyano, Alkoxycarbonyl mit 1 bis 4 Kohlenstoff-Atomen in der Alkyl-Struktureinheit oder Phenoxycarbonyl bezeichnet.

Ganz besonders bevorzugte Benzimidate der Formel (I) sind diejenigen, in denen
$R^1$ Trifluoromethyl oder Trifluoromethoxy ist,
X Fluor oder Chlor ist,
Z ein Schwefel-Atom ist,
Y Fluor ist,
n 0 oder 1 ist,
Q ein Sauerstoff-oder Schwefel-Atom ist und
$R^2$ Wasserstoff, Methyl, Ethyl, Phenyl, Acetyl oder Phenylthio ist.

Speziell seien die folgenden Verbindungen erwähnt:
S-Wasserstoff-N-(6-trifluoromethoxybenzothiazol-2-yl)-2,6-difluorobenzothioimidat;
Methyl-N-(6-trifluoromethoxybenzothiazol-2-yl)-2,6-difluorobenzimidat;
Ethyl-N-(6-trifluoromethoxybenzothiazol-2-yl)-2,6-difluorobenzimidat;
Isopropyl-N-(6-trifluoromethoxybenzothiazol-2-yl)-2,6-difluorobenzimidat;
Phenyl-N-(6-trifluoromethoxybenzothiazol-2-yl)-2,6-difluorobenzimidat und
S-Acetyl-N-(6-trifluoromethoxybenzothiazol-2-yl)-2,6-difluorobenzothioimidat.

Die Verbindungen der Formel (I) können beispielsweise mittels der folgenden Verfahren hergestellt werden.

Wenn N-(6-Trifluoromethoxybenzothiazol-2-yl)-2,6-difluorobenzimidoylchlorid und Ethanol als Ausgangsstoffe in Verfahren (a) eingesetzt werden, wird die Reaktion durch das folgende Schema veranschaulicht.

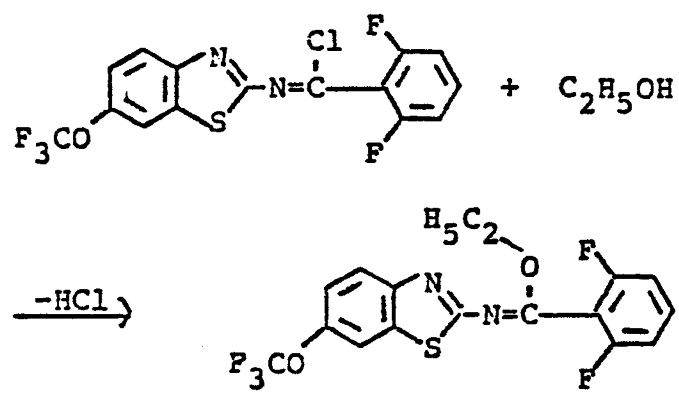


Wenn S-Wasserstoff-N-(6-trifluoromethoxybenzothiazol-2-yl)-2,6-difluorobenzothioimidat und Iodomethan als Ausgangsstoffe in Verfahren (b) eingesetzt werden, wird die Reaktion durch das folgende Schema veranschaulicht.

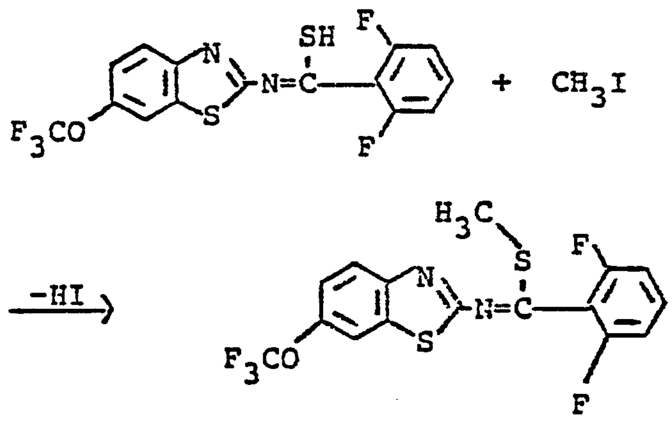


Wenn S-Wasserstoff-N-(6-trifluoromethoxybenzothiazol-2-yl)-2,6-difluorobenzothioimidat und Benzolsulfenylchlorid als Ausgangsstoffe in Verfahren (c) eingesetzt werden, wird die Reaktion durch das folgende Schema veranschaulicht.

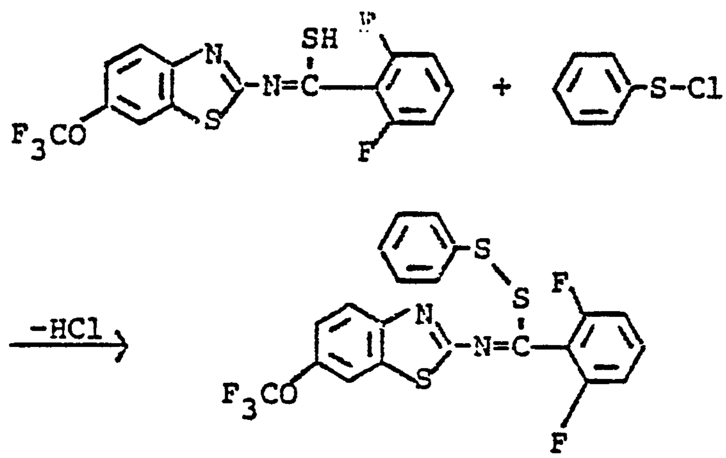


Wenn N-(6-Trifluoromethoxybenzothiazol-2-yl)-2,6-difluorobenzimidoylchlorid und Kaliumthiocyanat als Ausgangsstoffe in Verfahren (d) eingesetzt werden, wird die Reaktion durch das folgende Schema dargestellt.

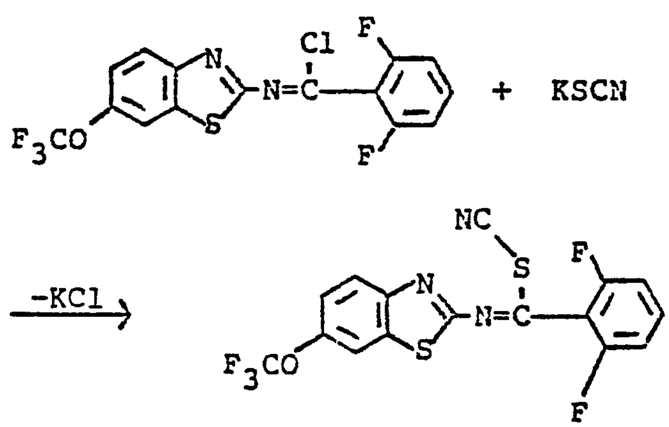


Wenn N-(6-Trifluoromethoxybenzothiazol-2-yl)-2,6-difluorobenzothioamidat-Kalium-Salz und Cyanbromid als Ausgangsstoffe in Verfahren (e) eingesetzt werden, wird die Reaktion durch das folgende Schema veranschaulicht.

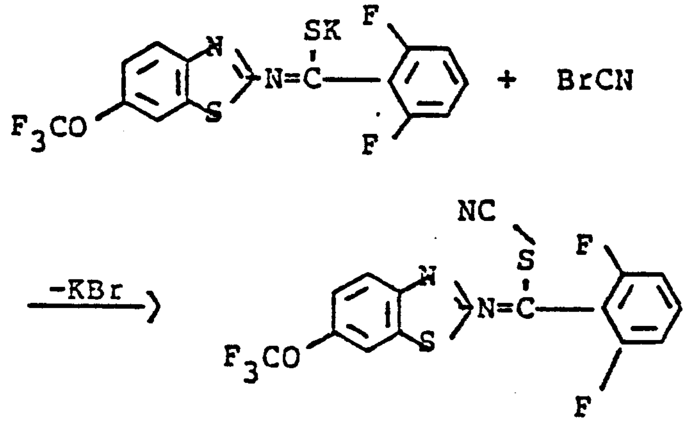


Wenn S-Methyl-N-(6-trifluoromethoxybenzothiazol-2-yl)-2,6-difluorobenzothioimidat und N,N-Dimethylethylendiamin als Ausgangsstoffe in Verfahren (f) eingesetzt werden, wird die Reaktion durch das folgende Schema veranschaulicht.

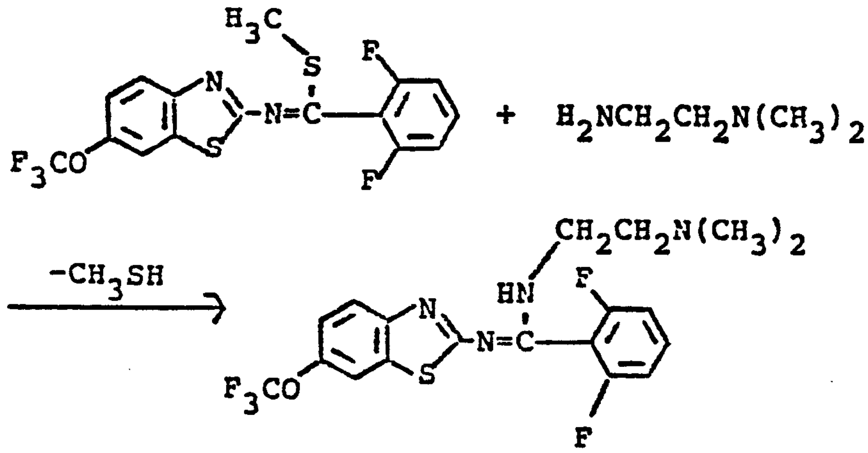


Die Verbindungen der Formel (II) sind neue Verbindungen und können im allgemeinen dadurch erhalten werden, daß eine Verbindung der Formel (IX)

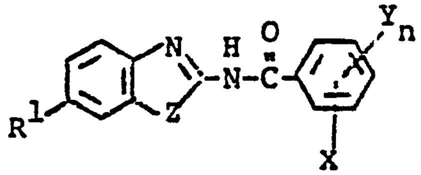
(IX)

in der

R', X, Y, Z und n die gleichen Bedeutungen wie oben angegeben haben,

mit Phosphorpentachlorid umgesetzt werden.

Die Verbindungen der Formel (IX) können nach bekannten Verfahren hergestellt werden (siehe z.B. Agric. Food Chem. 24 , 1976, 1065-1068).

Das Verfahren zur Herstellung der Verbindungen der Formel (II) kann zum Beispiel in der Weise praktisch durchgeführt werden, daß 1 mol der Verbindungen der Formel (IX) mit 1 bis etwa 1,2 mol Phosphorpentachlorid in Gegenwart eines inerten Lösungsmittels, beispielsweise eines gesättigten Kohlenwasserstoffs wie Benzol, Toluol, Xylol oder Hexan, eines chlorierten Kohlenwasserstoffs wie Chloroform oder eines Ethers wie Dioxan bei einer Temperatur von beispielsweise etwa 80°C bis etwa 150°C während einer Zeitspanne von etwa 0,1 h bis etwa 5 h umgesetzt wird.

Zu speziellen Beispielen für die Verbindungen der Formel (II) zählen

N-(6-Trifluoromethylbenzothiazol-2-yl)-2,6-difluorobenzimidoylchlorid,

N-(6-Trifluoromethoxybenzothiazol-2-yl)-2,6-difluorobenzimidoylchlorid,

N-(6-Trifluoromethylbenzothiazol-2-yl)-2-chlorobenzimidoylchlorid,
N-(6-Trifluoromethoxybenzothiazol-2-yl)-2-toluimidoylchlorid,
N-(6-Trifluoromethoxybenzothiazol-2-yl)-2-chlorobenzimidoylchlorid,
N-(6-Trifluoromethoxybenzothiazol-2-yl)-3,5-dichlorobenzimidoylchlorid,
N-(6-Trifluoromethoxybenzothiazol-2-yl)-2-chloro-6-fluorobenzimidoylchlorid,
N-(6-Trifluoromethoxybenzothiazol-2-yl)-2,4,6-trifluorobenzimidoylchlorid,
N-(6-Difluoromethoxybenzothiazol-2-yl)-2,6-difluorobenzimidoylchlorid,
N-[6-(1,1,2,2-Tetrafluoroethoxy)benzothiazol-2-yl]-2,6-difluorobenzimidoylchlorid,
N-[6-(1,1,2,3,3,3-Hexafluoropropoxy)benzothiazol-2-yl]-2,6-difluorobenzimidoylchlorid,
N-(6-Trifluoromethylthiobenzothiazol-2-yl)-2,6-difluorobenzimidoylchlorid,
N-(6-Trifluoromethylsulfonylbenzothiazol-2-yl)-2,6-difluorobenzimidoylchlorid und
N-(6-Chlorobenzothiazol-2-yl)-2,6-difluorobenzimidoylchlorid.

Die Verbindungen der Formel (III) sind auf dem Gebiet der organischen Chemie wohlbekannt, und zu speziellen Beispielen gehören Methanol, Ethanol, Propanol, Isopropanol, Methylmercaptan und Phenylmercaptan.

Die Verbindungen der Formel (IV), die Ausgangsstoffe in den Verfahren (b) und (c) sind, sind die mittels des Verfahrens (a) hergestellten Verbindungen.

Speziell die Verbindungen der Formel (IV) können dadurch hergestellt werden, daß die Verbindungen der Formel (II) mit Hydrogensulfid umgesetzt werden. Das entstehende Produkt weist Keto-Enol-Tautomerie entsprechend der nachstehenden Darstellung auf.

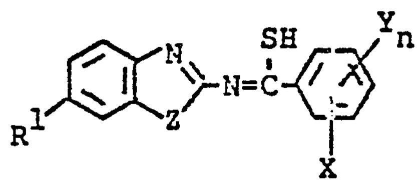

(Enol-Form)

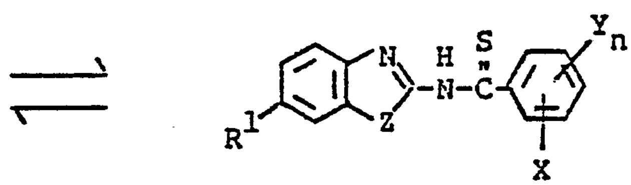

(Keto-Form)

Das Verfahren zur Herstellung der Verbindungen der Formel (IV) kann in der Weise durchgeführt werden, daß die Verbindungen der Formel (II) mit Hydrogensulfid in Gegenwart eines geeigneten inerten Lösungsmittels wie Benzol, Xylol, Toluol, Hexan, Chloroform und Dioxan bei etwa 60°C bis etwa 120°C umgesetzt werden, bis die Entwicklung von Hydrogenchlorid beendet ist, wonach die gewünschten Verbindungen der Formel (IV) erhalten werden.

In dem Verfahren (b) steht M vorzugsweise für ein Brom-Atom, ein Iod-Atom, $-OSO_2CH_3$ oder

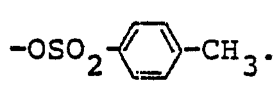

Die Verbindungen der Formel (V) sind bekannte Verbindungen. Einige dieser Verbindungen sind als Alkylierungsmittel gut bekannt. Spezielle Beispiele sind Iodomethan, Bromomethan, Iodoethan, 1-Iodopropan, 2-Iodopropan und Methyl-p-toluolsulfonat.

Die Verbindungen der Formel (VI) sind auf dem Gebiet der organischen Chemie wohlbekannt. Zu speziellen Beispielen zählen Methansulfenylchlorid, Ethansulfenylchlorid, Acetylchlorid, Benzolsulfenylchlorid und Propionylchlorid.

Kaliumthiocyanat ist ein spezielles Beispiel für das im Verfahren (d) verwendete Thiocyanat-Salz.

In dem Verfahren (e) können die Ausgangs-Verbindungen der Formel (VII) in einfacher Weise dadurch synthetisiert werden, daß die Verbindungen der Formel (IV) mit Alkali-oder Erdalkalimetall-Basen wie Kaliumhydroxid umgesetzt werden.

Die Ausgangs-Verbindungen der Formel (I-b) für das Verfahren (f) können in einfacher Weise mittels des Verfahrens (b) gewonnen werden, wie im Vorstehenden erwähnt wurde.

In gleicher Weise sind die als Ausgangsstoffe eingesetzten Verbindungen der Formel (VIII) auf dem Gebiet der organischen Chemie wohlbekannt. Spezielle Beispiele sind Ammoniak, Methylamin, Dimethylamin, N,N-Dimethylethylendiamin, Isopropylamin, tert-Butylamin, Anilin, 2-Ethoxyethylamin und N-Methylanilin.

In der Praxis des Verfahrens (a) können sämtliche inerten organischen Lösungsmittel als geeignete Verdünnungsmittel verwendet werden.

Zu geeigneten derartigen Verdünnungsmitteln zählen aliphatische, alicyclische und aromatische Kohlenwasserstoffe (die gegebenenfalls chloriert sein können), zum Beispiel Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether, zum Beispiel Diethylether, Methylethylether, Diisopropylether, Dibutylether, Dioxan und Tetrahydrofuran; Ketone, zum Beispiel Aceton, Methylethylketon, Methylisopropylketon und Methylisobutylketon; Nitrile, zum Beispiel Acetonitril, Propionitril und Acrylnitril; Ester, zum Beispiel Ethylacetat und Amylacetat; Säureamide, zum Beispiel Dimethylformamid und Dimethylacetamid; Sulfone und Sulfoxide, zum Beispiel Dimethylsulfoxid und Sulfolan; und Basen wie Pyridin.

Das Verfahren (a) kann auch in Gegenwart einer Base durchgeführt werden.

Derartige Basen können anorganische und organische Basen umfassen, die nicht mit den als Ausgangsstoffen eingesetzten Imidoylchloriden der Formel (II) reagieren oder mit diesen nur wenig unter speziellen Bedingungen reagieren, zum Beispiel Hydroxide, Carbonate, Hydrogencarbonate und Alkoholate von Alkalimetallen sowie tertiäre Amine, beispielsweise Triethylamin, Diethylanilin, Pyridin und Lutidin.

Das Verfahren (a) kann innerhalb eines breiten Temperatur-Bereichs durchgeführt werden, beispielsweise bei einer Temperatur zwischen etwa -20°C und etwa 100°C, vorzugsweise zwischen etwa 0°C und etwa 80°C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens (a) können die gewünschten Verbindungen der Formel (I) beispielsweise in der Weise erhalten werden, daß man 1 mol der Verbindungen der Formel (II) mit 1 bis etwa 1,5 mol, vorzugsweise etwa 1,2 mol, der Verbindungen der Formel (III) in Gegenwart des inerten Lösungsmittels und der Base umsetzt.

Bei der praktischen Durchführung des Verfahrens (b) sind Beispiele für geeignete Lösungsmittel inerte Lösungsmittel, beispielsweise Wasser, Alkohole wie Methanol, Ethanol, Isopropanol und Butanol, Säureamide wie Dimethylformamid und Dimethylacetamid, Sulfoxide wie Dimethylsulfoxid und Nitrile wie Acetonitril und Propionitril.

Das Verfahren (b) kann auch in Gegenwart einer Base durchgeführt werden. Zu solchen Basen zählen zum Beispiel die Hydroxide, Carbonate und Alkoholate von Alkalimetallen, die oben für das Verfahren (a) genannt wurden, sowie auch Alkalimetallhydride.

Das Verfahren (b) kann innerhalb eines breiten Temperatur-Bereichs durchgeführt werden, beispielsweise bei einer Temperatur zwischen etwa 0°C und etwa 100°C, vorzugsweise zwischen etwa 20°C und etwa 80°C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens (b) können die gewünschten Verbindungen beispielsweise dadurch erhalten werden, daß man 1 mol der Verbindungen der Formel (IV) mit 1 bis etwa 1,5 mol, vorzugsweise 1 bis etwa 1,2 mol, der Verbindungen der Formel (V) in Gegenwart des inerten Lösungsmittels und der Base umsetzt.

Bei der praktischen Durchführung des Verfahrens (c) sind geeignete Lösungsmittel inerte Lösungsmittel, beispielsweise Ether wie Diethylether, Methylethylether, Diisopropylether, Dibutylether, Dioxan und Tetrahydrofuran sowie Kohlenwasserstoffe wie Benzol, Toluol, Xylol, Hexan und Cyclohexan.

Das Verfahren (c) kann in Gegenwart einer Base durchgeführt werden. Pyridin, Lutidin, Triethylamin, Dimethylanilin und Natriumhydrid seien als Beispiele für eine solche Base angeführt.

Das Verfahren (c) kann innerhalb eines breiten Temperatur-Bereichs durchgeführt werden, beispielsweise bei einer Temperatur zwischen etwa -20°C und etwa 100°C, vorzugsweise zwischen etwa 0°C und etwa 50°C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens (c) können die gewünschten Verbindungen beispielsweise dadurch erhalten werden, daß man 1 mol der Verbindungen der Formel (IV) mit 1 bis etwa 1,5 mol, vorzugsweise 1 bis etwa 1,2 mol, der Verbindungen der Formel (VI) in Gegenwart des inerten Lösungsmittels und der Base umsetzt.

Die Verfahren (d) und (e) können in Gegenwart der gleichen inerten Lösungsmittel durchgeführt werden, wie sie in bezug auf das Verfahren (a) angegeben sind.

Die Verfahren (d) und (e) können innerhalb eines breiten Temperatur-Bereichs durchgeführt werden, beispielsweise bei einer Temperatur zwischen etwa -20°C und etwa 100°C, vorzugsweise zwischen etwa 0°C und etwa 80°C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens (f) verwendete geeignete Lösungsmittel sind inerte Lösungsmittel wie die oben beispielhaft genannten Alkohole, Ether, Benzol, Toluol und Xylol.

Das Verfahren (f) kann innerhalb eines breiten Temperatur-Bereichs durchgeführt werden, beispielsweise bei einer Temperatur zwischen Raumtemperatur und dem Siedepunkt der Mischung, vorzugsweise zwischen etwa 60°C und etwa 120°C. Zweckmäßigerweise wird die Reaktion unter Atmosphärendruck durchgeführt, jedoch ist es auch möglich, unter erhöhtem oder vermindertem Druck zu arbeiten.

Bei der praktischen Durchführung des Verfahrens (f) können die gewünschten Verbindungen beispielsweise dadurch erhalten werden, daß man 1 mol der Verbindung der Formel (Ib) mit 1 bis etwa 1,5 mol, vorzugsweise 1 bis etwa 1,2 mol, der Verbindungen der Formel (VIII) in dem inerten Lösungsmittel umsetzt.

Die Verbindungen der Formel (I) gemäß der vorliegenden Erfindung zeigen starke insektizide Aktivität und können aus diesem Grunde als Insektizide verwendet werden. Die aktiven Verbindungen der Formel (I) gemäß der vorliegenden Erfindung zeigen eine genaue herausragende Bekämpfungswirkung gegen Schädlinge, insbesondere gegen Larven von Schädlingen der Ordnung Lepidoptera, ohne daß sie bei Kulturpflanzen Phytotoxizität aufweisen. Die Verbindungen der vorliegenden Erfindung können auch zur Bekämpfung eines breiten Bereichs verschiedenartiger Schädlinge eingesetzt werden, beispielsweise gegen schädliche saugende und beißende Insekten, andere Pflanzenparasiten, Schädlinge an Lagergetreide und gesundheitsgefährdende Schädlinge, und können zur Bekämpfung und Ausrottung dieser Schädlinge angewandt werden.

Beispiele für die Schädlinge sind im Folgenden aufgeführt:

<u>Insekten der Ordnung Coleoptera</u>, Callosobruchus chinensis,
Sitophilus zeamais,
Tribolium castaneum,
Epilachna vigintioctomaculata,
Agriotes fuscicollis,
Anomala rufocuprea,
Leptinotarsa decemlineata,
Diabrotica spp.,
Monochamus alternatus,
Lissorhoptus oryzophilus und
Lyctus brunneus;

<u>Insekten der Ordnung Lepidoptera</u>, Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella,

Plutella maculipennis,
Heliothis virescens und
Phyllocnistis citrella;

Insekten der Ordnung Diptera, Musca domestica,
Aedes aegypti,
Hylemia platura,
Culex pipiens,
Anopheles sinensis und
Culex tritaeniorhynchus.

Milben, Tetranychus telarius,
Tetranychus urticae,
Panonychus citri,
Aculus pelekassi und
Tarsonemus spp..

Die erfindungsgemäßen aktiven Verbindungen eignen sich auch zur Bekämpfung solcher unerwünschter Schädlinge wie Insekten, Zecken und Milben auf dem Gebiet der bäuerlichen Tierhaltung und Tierzucht, und bessere Ergebnisse, beispielsweise höhere Milchausbeuten, höheres Gewicht, bessere Tierhäute, längeres Leben etc. lassen sich durch die Bekämpfung der Schädlinge erzielen.

Beispiele für solche Tierparasiten sind Insekten wie Gastrophilus spp. und Stomoxys spp. sowie Milben wie Soranithodorcs spp., Ixodes spp. und Boophilus spp..

Die aktiven Verbindungen können in gebräuchliche Formulierungen überführt werden, etwa Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, farbgebende Mittel, etwa anorganische Pigmente wie beispielsweise Eisenoxid, Titanoxid und Preußisch Blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in ihren handelsüblichen Formulierungen oder den aus diesen Formulierungen hergestellten Anwendungsformen im Gemisch mit anderen aktiven Verbindungen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die aktiven Verbindungen gemäß der vorliegenden Erfindung können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen, die die Wirkung der aktiven Verbindungen steigern, ohne daß es für das zugesetzte synergistische Mittel erforderlich ist, selbst aktiv zu sein.

Der Gehalt der aktiven Verbindung in den aus den im Handel erhältlichen Formulierungen hergestellten Anwendungsformen kann innerhalb weiter Grenzen variieren. Die Konzentration der aktiven Verbindung in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die aktiven Verbindungen durch eine hervorragende Rückstandswirkung auf Holz und Ton sowie eine gute Beständigkeit gegen Alkali auf gekalkten Unterlagen aus.

Die folgenden Beispiele erläutern die vorliegende Erfindung im einzelnen. Die vorliegende Erfindung ist jedoch nicht auf diese Beispiele beschränkt.

## Beispiel 1

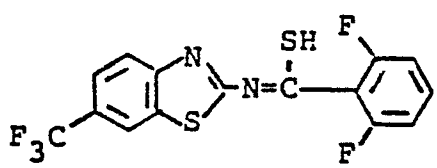

(Enol-Form)

(Verbindung Nr. 1)

Eine Mischung aus N-(6-Trifluoromethylbenzothiazol-2-yl)-2,6-difluorobenzamid (3,56 g), Phosphorpentachlorid (2,2 g) und trockenem Toluol (20 ml) wurde 5 min bei 95°C bis 100°C gerührt, und Hydrogensulfid wurde bei der gleichen Temperatur eingeleitet. Als die Erzeugung von Hydrogenchlorid im wesentlichen beendet war, wurde die Mischung auf Raumtemperatur gekühlt und filtriert. Die niedrigsiedenden Substanzen wurden aus dem Filtrat unter vermindertem Druck abgedampft, und der Rückstand wurde mit einer 5-proz. wäßrigen Lösung von Kaliumhydroxid (50 ml) vermischt. Die Mischung wurde filtriert, und die wäßrige Lösung wurde mit Toluol (50 ml) gewaschen. Die wäßrige Schicht wurde abgetrennt, mit Essigsäure angesäuert und dann mit Toluol (50 ml) extrahiert. Nach dem Waschen mit Wasser wurde der Extrakt über wasserfreiem Natriumsulfat getrocknet. Das Toluol wurde abdestilliert, und der Rückstand wurde aus Toluol-Hexan umkristallisiert, wonach das gewünschte SH-N-(6-Trifluoromethylbenzothiazol-2-yl)-2,6-difluorobenzothioimidat (1,3 g; Schmp. 236-238°C) erhalten wurde.

Die vorstehende Verbindung Nr. 1 kann auch in der folgenden Keto-Form beschrieben werden:

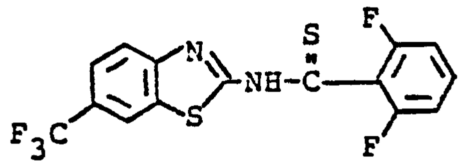

(Keto-Form) .

Beispiel 2

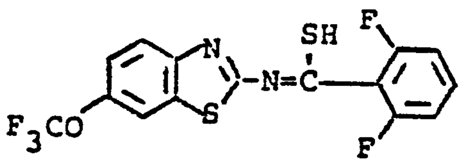

(Enol-Form)

(Verbindung Nr. 2)

N-(6-Trifluoromethoxybenzothiazol-2-yl)-2,6-difluorobenzimidoylchlorid (3,91 g) wurde in trockenem Toluol (20 ml) gelöst, und Hydrogen wurde bei 90°C bis 100°C eingeleitet, bis die Entwicklung von Hydrogenchlorid aufhörte. Toluol wurde unter vermindertem Druck abdestilliert, und der Rückstand wurde aus Toluol-Hexan umkristallisiert, wonach das gewünschte SH-N-(6-Trifluoromethoxybenzothiazol-2-yl)-2,6-difluorobenzothioimidat (3,2 g; Schmp. 214,5-215,5°C) erhalten wurde.

Die vorstehende Verbindung Nr. 2 hat die folgende Keto-Form, die im Gemisch mit der Enol-Form anfällt:

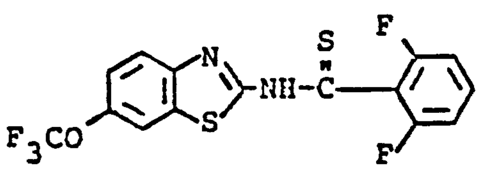

(Keto-Form) .

Beispiel 3

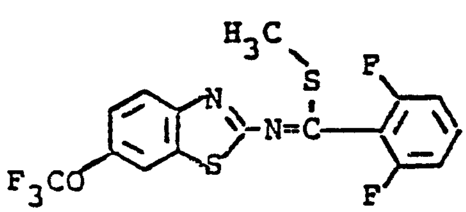

(Verbindung Nr. 3)

SH-N-(6-Trifluoromethoxybenzothiazol-2-yl)-2,6-difluorobenzothioimidat (3,89 g) wurde in Ethanol (30 ml) gelöst, und Natriumhydrid (0,3 g) wurde hinzugegeben. Die Mischung wurde gerührt, und Iodomethan (1,7 g) wurde der Lösung zugesetzt. Die Mischung wurde 1 h bei 30°C und dann 5 h bei 50°C bis 55°C gerührt. Niedrigsiedende Substanzen wurden unter vermindertem Druck abdestilliert, und Toluol (80 ml) und Wasser (50 ml) wurden zu dem Rückstand hinzugefügt. Nachdem der Rückstand sich in zwei Schichten getrennt hatte, wurde die Toluol-Schicht mit einer 5-proz. wäßrigen Kaliumhydroxid-Lösung und dann mit Wasser gewaschen und über wasserfreiem Natriumsulfat getrocknet. Das Toluol wurde unter vermindertem Druck abdestilliert, wonach das gewünschte S-Methyl-N-(6-trifluoromethoxybenzothiazol-2-yl)-2,6-difluorobenzothioimidat (3,4 g; $n_{D}^{20}$ = 1,6080) erhalten wurde.

Beispiel 4

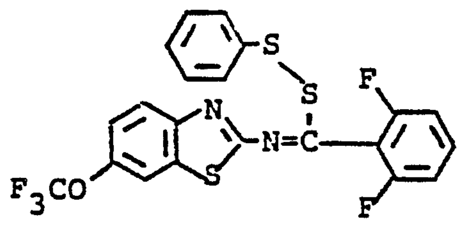

(Verbindung Nr. 4)

Zu einer Lösung aus SH-N-(6-Trifluoromethoxybenzothiazol2-yl)-2,6-difluorobenzothioimidat (l,l g), Toluol (20 ml) und Triethylamin (0,27 g) wurde tropfenweise Benzolsulfenylchlorid (0,38 g) bei 10°C hinzugefügt. Die Mischung wurde 1 d bei Raumtemperatur gerührt. Die Reaktionsmischung wurde mit 1-proz. wäßrigem HCl, einer gesättigten wäßrigen Lösung von Natriumhydrogencarbonat und Wasser in dieser Reihenfolge gewaschen und über wasserfreiem Natriumsulfat getrocknet. Das Toluol wurde unter vermindertem Druck abdestilliert, und der Rückstand wurde an einer Silicagel-Säule mit Toluol als Entwicklungslösung gereinigt, wonach das gewünschte S-Phenylmercapto-N-(6-trifluoromethoxybenzothiazol-2-yl)-2,6-difluorobenzothioimidat (0,85 g; $n_{D}^{20}$ = 1,6485) erhalten wurde.

Beispiel 5

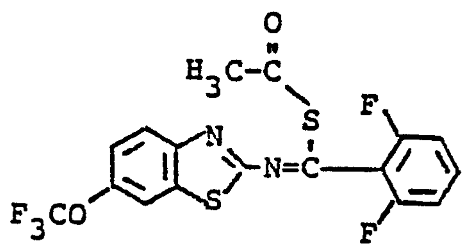

(Verbindung Nr. 5)

Acetylchlorid (0,21 g) wurde tropfenweise bei -10°C bis O°C zu einer Lösung aus SH-N-(6-Trifluoromethoxybenzothiazol-2-yl)-2,6-difluorobenzothioimidat (l,l g), Toluol (20 ml) und Triethylamin (0,27 g) hinzugefügt. Die Mischung wurde 3 h bei Raumtemperatur gerührt, und die Reaktionsmischung wurde mit Wasser und dann mit einer gesättigten wäßrigen Natriumchlorid-Lösung gewaschen und über wasserfreiem Natriumsulfat getrocknet. Das Toluol wurde unter vermindertem Druck abdestilliert, und der

Rückstand wurde an einer Silicagel-Säule unter Verwendung von Toluol gereinigt und aus N-Hexan umkristallisiert, wonach das gewünschte S-Acetyl-N-(6-trifluoromethoxybenzothiazol-2-yl)-2,6-difluorobenzothioimidat (0,1 g; Schmp. 121-122°C) in Form karminroter Kristalle erhalten wurde.

Beispiel 6

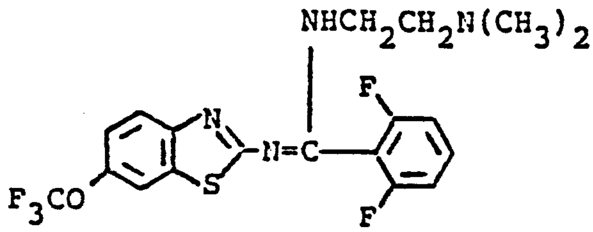

(Verbindung Nr. 6)

Eine Mischung aus S-Methyl-N-(6-trifluoromethoxybenzothiazol-2-yl)-2,6-difluorobenzothioimidat (1,8 g), Ethanol (10 ml) und N,N-Dimethylethylendiamin (0,4 g) wurde 8 h unter Rückfluß erhitzt. Das Ethanol wurde abdestilliert. Der Rückstand wurde mit Wasser (20 ml) gut vermischt, und der feste Stoff wurde durch Filtration gesammelt und mit gekühltem Wasser gewaschen. Der getrocknete feste Stoff wurde aus Toluol-Hexan umkristallisiert, wonach das gewünschte N-2-(Dimethylamino)-ethyl-N'-(6-trifluoromethoxybenzothiazol-2-yl)-2,6-difluorobenzamidin (1 g; Schmp. 132-133°C) erhalten wurde.

Beispiel 7

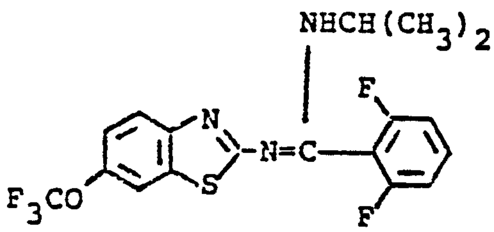

(Verbindung Nr. 7)

Zu einer Lösung aus N-(6-Trifluoromethoxybenzothiazol-2-yl)-2,6-difluorobenzimidoylchlorid (1,39 g) und Toluol (30 ml) wurde tropfenweise Isopropylamin (0,46 g) bei 0°C hinzugefügt. Die Mischung wurde 3 h bei Raumtemperatur gerührt. Die Reaktionsmischung wurde mit Wasser gewaschen und über wasserfreiem Natriumsulfat getrocknet. Das Toluol wurde abdestilliert, und der Rückstand wurde aus Hexan umkristallisiert, wonach das gewünschte N-Isopropyl-N'-(6-trifluoromethoxybenzothiazol-2-yl)-2,6-difluorobenzamidin (0,8 g; Schmp. 89-90°C) erhalten wurde.

Beispiel 8

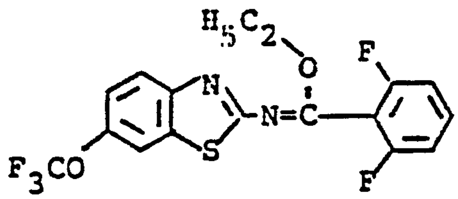

(Verbindung Nr. 8)

N-(6-Trifluoromethoxybenzothiazol-2-yl)-2,6-difluorobenzimidoylchlorid (1,42 g) wurde tropfenweise zu einer Mischung aus Ethanol (0,2 g), Triethylamin (0,4 g) und Toluol (20 ml) bei 20°C bis 25°C hinzugefügt. Die Reaktionsmischung wurde 1 h bei 35°C bis 45°C gerührt, gekühlt, mit 1-proz. HCl und Wasser in dieser Reihenfolge gewaschen und über wasserfreiem Natriumsulfat getrocknet. Das Toluol wurde unter vermindertem Druck abdestilliert, wonach das gewünschte Ethyl-N-(6-trifluoromethoxybenzothiazol-2-yl)-2,6-difluorobenzimidat (1,2 g; $n_D^{20}$ = 1,5657) erhalten wurde.

Beispiel 9

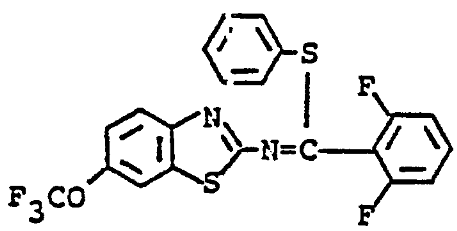

(Verbindung Nr. 9)

Thiophenol (0,4 g wurde in Toluol (30 ml) gelöst, und Natriumhydrid (0,09 g) wurde zugegeben. Nach beendeter Wasserstoff-Entwicklung wurde eine Lösung aus N-(6-Trifluoromethoxybenzothiazol-2-yl)-2,6-difluorobenzimidoylchlorid (1,42 g) und Toluol (15 ml) bei 10°C tropfenweise hinzugefügt. Die Mischung wurde 5 h bei Raumtemperatur gerührt. Die Reaktionsmischung wurde mit Wasser, mit einer 5-proz. wäßrigen Kaliumhydroxid-Lösung und mit Wasser in dieser Reihenfolge gewaschen und über wasserfreiem Natriumsulfat getrocknet. Das Toluol wurde abdestilliert, und der Rückstand wurde aus Toluol-Hexan umkristallisiert, wonach das gewünschte S-Phenyl-N-(6-trifluoromethoxybenzothiazol-2-yl)-2,6-difluorobenzothioimidat (0,2 g; Schmp. 106-107°C) erhalten wurde.

Verbindungen, die nach den Arbeitsmethoden der vorhergehenden Beispiele synthetisiert wurden, sind in Tabelle 1 aufgeführt.

Tabelle 1

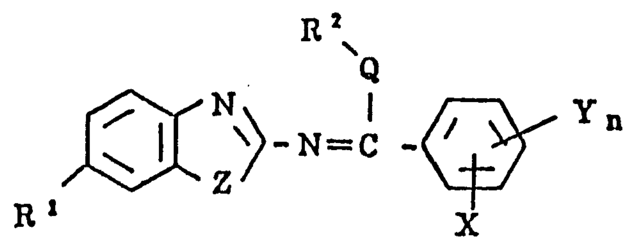

| Verbindung Nr. | $R^1$ | Z | $-Q-R^2$ | X , $Y_n$ | |
|---|---|---|---|---|---|
| 10 | $-OCF_3$ | S | $-OH$ | $3,5-Cl_2$ | Schmp. 215–216°C |
| 11 | $-OCF_3$ | S | $-SH$ | 2-Cl | Schmp. 196–198°C |
| 12 | $-OCF_3$ | S | $-SH$ | $3,5-Cl_2$ | Schmp. 205–206°C |
| 13 | Cl | S | $-OCH_3$ | $2,6-F_2$ | Schmp. 86–87°C |
| 14 | $-CF_3$ | S | $-OCH_3$ | $2,6-F_2$ | $n_D^{20}$ 1.5698 |

- wird fortgesetzt -

Tabelle 1 Fortsetzung

| | | | | | |
|---|---|---|---|---|---|
| 15 | $-CF_3$ | S | $-OC_2H_5$ | 2,6-$F_2$ | $n_D^{20}$ 1.5752 |
| 16 | $-CF_3$ | S | $-OCH(CH_3)_2$ | 2,6-$F_2$ | $n_D^{20}$ 1.5527 |
| 17 | $-CF_3$ | S | $-OCH_2CH=CH_2$ | 2,6-$F_2$ | $n_D^{20}$ 1.5652 |
| 18 | $-CF_3$ | S | $-OCH_3$ | 2-Cl | $n_D^{25}$ 1.5866 |
| 19 | $-CF_3$ | S | $-OCH(CH_3)_2$ | 2-Cl | $n_D^{25}$ 1.5718 |
| 20 | $-OCF_3$ | S | $-OCH_3$ | 2-$CH_3$ | $n_D^{20}$ 1.5718 |
| 21 | $-OCF_3$ | S | $-OCH(CH_3)_2$ | 2-$CH_3$ | $n_D^{20}$ 1.5564 |
| 22 | $-OCF_3$ | S | $-OC_2H_5$ | 2-Cl | $n_D^{20}$ 1.5948 |
| 23 | $-OCF_3$ | S | $-OCH(CH_3)_2$ | 2-Cl | |
| 24 | $-OCF_3$ | S | $-OC_2H_5$ | 2-Cl, 6-F | $n_D^{25}$ 1.5657 |
| 25 | $-OCF_3$ | S | $-OCH_3$ | 2,6-$F_2$ | $n_D^{20}$ 1.5590 |

- wird fortgesetzt -

Tabelle 1 Fortsetzung

| | | | | | |
|---|---|---|---|---|---|
| 26 | $-OCF_3$ | S | $-OCH(CH_3)_2$ | 2,6-$F_2$ | $n_D^{20}$ 1.5402 |
| 27 | $-OCF_3$ | S | $-OC_3H_7$-n | 2,6-$F_2$ | $n_D^{20}$ 1.5488 |
| 28 | $-OCF_3$ | S | $-OC_4H_9$-n | 2,6-$F_2$ | $n_D^{20}$ 1.5448 |
| 29 | $-OCF_3$ | S | -O- 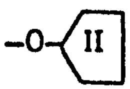 | 2,6-$F_2$ | Schmp. 87–88°C |
| 30 | $-OCF_3$ | S | $-OCH_2CH_2OCH_3$ | 2,6-$F_2$ | |
| 31 | $-OCF_3$ | S | $-OCH_2CH_2OC_2H_5$ | 2,6-$F_2$ | $n_D^{20}$ 1.5460 |
| 32 | $-OCF_3$ | S | $-OCH_2CH_2SC_2H_5$ | 2,6-$F_2$ | $n_D^{20}$ 1.5600 |
| 33 | $-OCF_3$ | S | $-OCH_2CH_2O$- 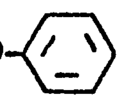 | 2,6-$F_2$ | Schmp. 104–105°C |
| 34 | $-OCF_3$ | S | $-OCH_2CH_2N(CH_3)_2$ | 2,6-$F_2$ | $n_D^{20}$ 1.5463 |
| 35 | $-OCF_3$ | S | $-OCH_2CH_2N$ 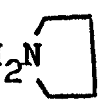 | 2,6-$F_2$ | |

- wird fortgesetzt -

<u>Tabelle 1 Fortsetzung</u>

| | | | | | |
|---|---|---|---|---|---|
| 36 | $-OCF_3$ | S | $-O(CH_2CH_2O)_2C_2H_5$ | $2,6-F_2$ | $n_D^{20}$ 1.5405 |
| 37 | $-OCF_3$ | S | $-OCH_2CF_3$ | $2,6-F_2$ | $n_D^{20}$ 1.5235 |
| 38 | $-OCF_3$ | S | $-OCH_2CCl_3$ | $2,6-F_2$ | |
| 39 | $-OCF_3$ | S | $-OCH_2CH{=}CH_2$ | $2,6-F_2$ | $n_D^{20}$ 1.5575 |
| 40 | $-OCF_3$ | S | $-OCH_2C{\equiv}CH$ | $2,6-F_2$ | $n_D^{20}$ 1.5633 |
| 41 | $-OCF_3$ | S | $-OCH_2-$ 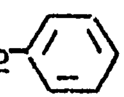 | $2,6-F_2$ | $n_D^{20}$ 1.5784 |
| 42 | $-OCF_3$ | S | $-O-$ 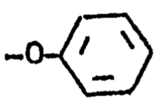 | $2,6-F_2$ | $n_D^{20}$ 1.5905 |
| 43 | $-OCF_3$ | S | $-O-C_4H_9$-tert | $2,6-F_2$ | Schmp. 102-103°C |
| 44 | $-OCF_3$ | S | $-S-SCH_3$ | $2,6-F_2$ | $n_D^{20}$ 1.6343 |
| 45 | $-OCF_3$ | S | $-S-SC_2H_5$ | $2,6-F_2$ | |

- wird fortgesetzt -

Tabelle 1 Fortsetzung

| | | | | | |
|---|---|---|---|---|---|
| 46 | $-OCF_3$ | S | $-S-SC_3H_7-n$ | 2,6-$F_2$ | |
| 47 | $-OCF_3$ | S | $-S-SC(CH_3)_3$ | 2,6-$F_2$ | |
| 48 | $-OCF_3$ | S | -S-S- 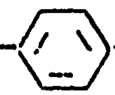 $-CH_3$ | 2,6-$F_2$ | |
| 49 | $-OCF_3$ | S | -S-S- 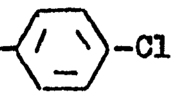 -Cl | 2,6-$F_2$ | |
| 50 | $-OCF_3$ | S | -SCN | 2,6-$F_2$ | Schmp. 186-188°C |
| 51 | $-CF_3$ | S | $-NH_2$ | 2,6-$F_2$ | Schmp. 175-177°C |
| 52 | $-OCF_3$ | S | $-NH_2$ | 2,6-$F_2$ | Schmp. 129-131°C |
| 53 | $-OCF_3$ | S | $-NHCH_3$ | 2,6-$F_2$ | |
| 54 | $-OCF_3$ | S | $-N(C_2H_5)_2$ | 2,6-$F_2$ | $n_D^{20}$ 1.5780 |
| 55 | $-OCF_3$ | S | -NH- 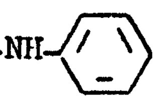 | 2,6-$F_2$ | Schmp. 161-162.5°C |

- wird fortgesetzt -

Tabelle 1 Fortsetzung

| | | | | | |
|---|---|---|---|---|---|
| 56 | $-OCF_3$ | S | $-NHCH_2CH_2OC_2H_5$ | 2,6-$F_2$ | |
| 57 | $-OCF_3$ | S | $-NHCH_2CH_2SC_2H_5$ | 2,6-$F_2$ | Schmp. 82-83°C |
| 58 | -Cl | S | $-OCH(CH_3)_2$ | 2,6-$F_2$ | |
| 59 | $-OCHF_2$ | S | $-OC_2H_5$ | 2,6-$F_2$ | |
| 60 | $-OCF_2CHF_2$ | S | $-OCH_3$ | 2,6-$F_2$ | |
| 61 | $-OCF_2CHF_2$ | S | $-OC_2H_5$ | 2,6-$F_2$ | |
| 62 | $-OCF_2CHF_2$ | S | $-OCH(CH_3)_2$ | 2,6-$F_2$ | |
| 63 | $-OCF_2CHFCF_3$ | S | $-OCH_3$ | 2,6-$F_2$ | $n_D^{25}$ 1.5254 |
| 64 | $-OCF_2CHFCF_3$ | S | $-OC_2H_5$ | 2,6-$F_2$ | |
| 65 | $-OCF_2CHFCF_3$ | S | $-OCH(CH_3)_2$ | 2,6-F | |
| 66 | $-SCF_3$ | S | $-OCH_3$ | 2,6-$F_2$ | $n_D^{25}$ 1.5782 |

- wird fortgesetzt -

Tabelle 1 Fortsetzung

| | | | | | |
|---|---|---|---|---|---|
| 67 | $-SCF_3$ | S | $-OC_2H_5$ | 2,6-$F_2$ | |
| 68 | $-SCF_3$ | S | $-OCH(CH_3)_2$ | 2,6-$F_2$ | |
| 69 | $-\overset{O}{\overset{\|}{\underset{\underset{O}{\|}}{S}}}-CF_3$ | S | $-OCH_3$ | 2,6-$F_2$ | |
| 70 | $-\overset{O}{\overset{\|}{S}}-CF_3$ | S | $-SH$ | 2,6-$F_2$ | |
| 71 | $-OCF_3$ | O | $-SH$ | 2,6-$F_2$ | |
| 72 | $-OCF_3$ | O | $-OC_2H_5$ | 2,6-$F_2$ | |
| 73 | $-OCF_3$ | S | $-SH$ | 2,4,6-$F_3$ | |
| 74 | $-OCF_2CHF_2$ | S | $-SH$ | 2,6-$F_2$ | Schmp.197–198°C |

Beispiel 10

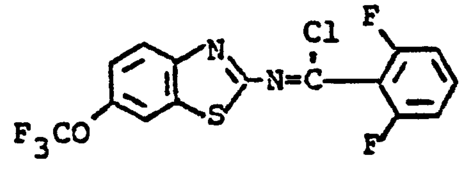

(Verbindung Nr. II-1)

Eine Mischung aus N-(6-Trifluoromethoxybenzothiazol-2-yl)-2,6-difluorobenzamid (37,2 g), trockenem Toluol (200 ml) und Phorphorpentachlorid (23 g) wurde bei 90°C gerührt. Die Hydrogenchlorid-Entwicklung war heftig, und nachdem sich eine klare Lösung gebildet hatte, wurde 5 min bei 100°C weitergerührt. Die Lösung wurde auf Raumtemperatur abgekühlt und filtriert, um Spuren unlöslicher, als Nebenprodukte gebildeter Stoffe zu entfernen. Das Filtrat wurde unter vermindertem Druck zur Trockne eingedampft. Hexan wurde zugegeben, und die Mischung wurde erhitzt und filtriert. Das Filtrat wurde gekühlt, und die entstandenen Kristalle wurden gesammelt, wonach das gewünschte N-(6-Trifluoromethoxybenzothiazol-2-yl)-2,6-difluorobenzimidoylchlorid (22 g; Schmp. 57,5-58,5°C) erhalten wurde.

Typische Beispiele für die Verbindung der Formel (II), die nach der gleichen Methode wie in Beispiel 10 synthetisiert worden waren, sind in der nachstehenden Tabelle 2 aufgeführt.

Tabelle 2

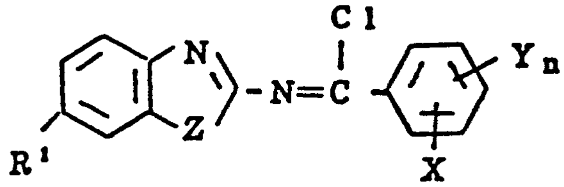

| Verbindung Nr. | $R^1$ | Z | X, $Y_n$ | |
|---|---|---|---|---|
| II-2 | $-CF_3$ | S | 2-Cl | $n_D^{30}$ 1.6172 |
| II-3 | $-CF_3$ | S | 2,6-$F_2$ | $n_D^{20}$ 1.5937 |
| II-4 | $-OCF_3$ | S | 2-$CH_3$ | Schmp. 73-74°C |
| II-5 | $-OCF_3$ | S | 2-Cl | Schmp. 78-79°C |
| II-6 | $-OCF_3$ | S | 3,5-$Cl_2$ | Schmp. 124-129°C |
| II-7 | $-OCF_3$ | S | 2-Cl, 6-F | $n_D^{29}$ 1.5912 |
| II-8 | $-OCF_3$ | S | 2,4,6-$F_3$ | |
| II-9 | $-OCHF_2$ | S | 2,6-$F_2$ | |
| II-10 | $-OCF_2CHF_2$ | S | 2,6-$F_2$ | Schmp. 81-82°C |
| II-11 | $-OCF_2CHFCF_3$ | S | 2,6-$F_2$ | $n_D^{33}$ 1.5550 |
| II-12 | $-SCF_3$ | S | 2,6-$F_2$ | $n_D^{33}$ 1.6164 |
| II-13 | O<br>$-S-CF_3$<br>O | S | 2,6-$F_2$ | |
| II-14 | -Cl | S | 2,6-$F_2$ | Schmp. 103-104°C |

<u>Beispiel für die Herstellung des Ausgangsstoffes der Formel IX:</u>

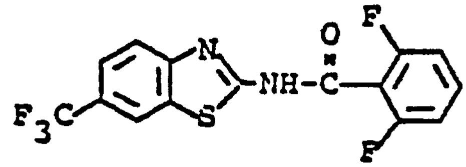

Bei 0°C bis 5°C wurde 2,6-Difluorobenzoylchlorid (1,77 g) tropfenweise zu einer Lösung aus 2-Amino-6-trifluoromethylbenzothiazol (2,18 g), Tetrahydrofuran (30 ml) und Triethylamin (1,1 g) hinzugefügt. Die Reaktionsmischung wurde 5 h bei 30°C bis 40°C gerührt, und dann wurde das Tetrahydrofuran abgedampft. Der feste Reückstand wurde mit Wasser gewaschen und aus Ethanol umkristallisiert, wonach das gewünschte N-(6-Trifluoromethylbenzothiazol-2-yl)-2,6-difluorobenzamid (2,8 g; Schmp. 256-257°C) erhalten wurde.

Biologische Tests

Vergleichsverbindung A:

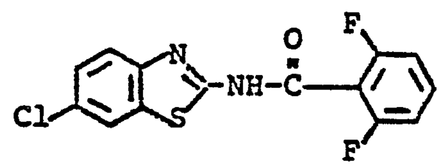

(die in J. Agr. Food Chem. 24 , 1976, 1065-1068, beschriebene Verbindung).

Beispiel 11

Test mit Larven von Spodoptera litura:

Herstellung einer Test-Chemikalie:

Lösungsmittel: 3 Gew.-Teile Dimethylformamid
Emulgator: 1 Gew.-Teil Polyoxyethylen-alkylphenylether

Zur Herstellung einer geeigneten Formulierung der aktiven Verbindung wurde 1 Gew.-Teil der betreffenden aktiven Verbindungen mit dem Emulgator und dem Lösungsmittel in den oben angegebenen Mengen vermischt, und die erhaltene Mischung wurde mit Wasser auf eine vorher festgesetzte Konzentration verdünnt.

Test-Verfahren:

Kohlblätter wurden in die wäßrige Verdünnung mit der vorbestimmten Wirkstoff-Konzentration getaucht, an der Luft getrocknet und in Petrischalen von 9 cm Durchmesser gelegt. 10 Larven im 3. Entwicklungsstadium von Spodoptera litura wurden in die Schale eingesetzt. Die Schalen wurden dann in einen auf 28°C gehaltenen Inkubator gestellt, und 7 d später wurde das Vernichtungsverhältnis berechnet.

Die Ergebnisse typischer Beispiele sind in Tabelle 3 aufgeführt.

Tabelle 3

| Verbindung Nr. | Wirkstoff-Konzentration ppm | Vernichtungs-verhältnis % |
|---|---|---|
| 1 | 8 | 100 |
| 2 | 8 | 100 |
| 3 | 8 | 100 |
| 4 | 8 | 100 |
| 5 | 8 | 100 |
| 8 | 8 | 100 |
| 9 | 8 | 100 |
| Vergleichs-Verbindung A | 40 | 80 |
| | 8 | 0 |

Beispiel 12

Test mit Larven von Plutella maculipennis:

Test Verfahren:

Kohlblätter wurden in eine wäßrige Verdünnung der aktiven Verbindung mit einer vorbestimmten Konzentration, die wie in Beispiel 11 hergestellt worden war, getaucht, an der Luft getrocknet und in eine Petri-Schale von 9 cm Durchmesser gelegt. Zehn Larven von Plutella maculipennis im zweiten Entwicklungsstadium wurden in der Schale ausgesetzt, und die Schalen wurden in einen auf 23°C gehaltenen Inkubator gestellt. 7 d später wurde das Vernichtungsverhältnis berechnet.

Die Ergebnisse typischer Beispiele sind in Tabelle 4 aufgeführt.

Tabelle 4

| Verbindung Nr. | Wirkstoff-Konzentration ppm | Vernichtungsverhältnis % |
|---|---|---|
| 1 | 40 | 100 |
| 2 | 40 | 100 |
| 3 | 40 | 100 |
| 5 | 40 | 100 |
| 8 | 40 | 100 |
| Vergleichs-Verbindung A | 200 | 100 |
| | 40 | 0 |

Beispiel 13

Test gegen Tetranychus urticae (Sprühtest):

Test-Verfahren:

50 bis 100 ausgewachsene Exemplare von Tetranychus urticae, die gegen Organophosphor-Mittel resistent waren, wurden auf den Blättern von Vits-Bohnen im Stadium der Entwicklung von zwei Hauptblättern ausgesetzt, die in Töpfen mit einem Durchmesser von 9 cm gezogen worden waren. 2 Tage später wurde eine nach dem gleichen Verfahren wie in Beispiel 11 hergestellte wäßrige Verdünnung der aktiven Verbindung mit festgelegter Konzentration in einer Menge von 20 ml je Topf auf die Blätter gesprüht. Die Töpfe wurden in ein Gewächshaus gestellt, und die Bekampfungswirkung der aktiven Verbindung wurde 10 Tage danach bewertet und durch die folgenden Indices bezeichnet.

3: Anteil der überlebenden ausgewachsenen Insekten: 0 %;

2: Anteil der überlebenden ausgewachsenen Insekten: 0 % bis 5 %;

1: Anteil der überlebenden ausgewachsenen Insekten: 5 % bis 50 %, bezogen auf die Zahl der Fälle in einem nicht behandelten Topf;

0: Anteil der überlebenden ausgewachsenen Insekten: mehr als 50 %, bezogen auf die Zahl der Fälle in einem nicht behandelten Topf.

Die Ergebnisse sind in Tabelle 5 aufgeführt.

Tabelle 5

| Verbindung Nr. | Wirkstoff-Konzentration ppm | Bekämpfungs-Index |
|---|---|---|
| 5 | 200 | 3 |
| 6 | 200 | 3 |
| Vergleichs-Verbindung A | 1000 | 0 |

## Ansprüche

1. Benzimidate der Formel (I)

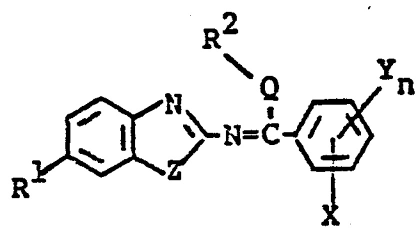

(I)

in der

$R^1$ Halogen, Halogenoalkyl, Halogenoalkoxy, Halogenoalkylthio, Halogenoalkylsulfinyl oder Halogenoalkylsulfonyl bezeichnet,

X Halogen oder Alkyl bezeichnet,

Z Sauerstoff oder Schwefel bezeichnet,

Y Halogen oder Alkyl bezeichnet,

n 0, 1 oder 2 ist,

Q Sauerstoff, Schwefel oder die Gruppe $>$N-$R^3$ bezeichnet, in der

$R^3$ Wasserstoff oder Alkyl bezeichnet, und

$R^2$ Wasserstoff, Alkyl, Halogenoalkyl, Alkoxyalkyl, Alkylthioalkyl, N,N-disubstituiertes Aminoalkyl, Aralkyl, Aryloxyalkyl, Arylthioalkyl, Cycloalkyl, Alkenyl, Alkinyl, Aryl, Alkylthio, Aralkylthio, Arylthio, Alkylcarbonyl, Alkoxycarbonyl, Aryloxycarbonyl oder Cyano bezeichnet.

2. Verbindungen der Formel (I) nach Anspruch 1, dadurch gekennzeichnet, daß

$R^1$ Fluor, Chlor, $C_1$-$C_2$-Fluoroalkyl, $C_1$-$C_2$-Fluoroalkoxy, $C_1$-$C_2$-Fluoroalkylthio, $C_1$-$C_2$-Fluoroalkylsulfinyl oder $C_1$-$C_2$-Fluoroalkylsulfonyl bezeichnet,

X Fluor, Chlor oder Methyl bezeichnet,

Z ein Schwefel-Atom bezeichnet,

Y Fluor, Chlor oder Methyl bezeichnet,

n 0, 1 oder 2 ist,

Q ein Sauerstoff-oder Schwefel-Atom oder die Gruppe $>$N-$R^3$, in der $R^3$ ein Wasserstoff-Atom oder $C_1$-$C_4$-Alkyl ist, bezeichnet und

$R^2$ Wasserstoff, $C_1$-$C_4$-Alkyl, $C_2$-$C_2$-Alkenyl, $C_2$-$C_3$-Alkinyl, Phenyl, $C_5$ -$C_6$-Cycloalkyl, Alkoxyalkyl mit insgesamt 2 bis 4 Kohlenstoff-Atomen, Alkylthioalkyl mit insgesamt 2 bis 4 Kohlenstoff-Atomen, Phenoxy-$C_1$-$C_2$-alkyl, Phenylthio-$C_1$-$C_2$-alkyl, Benzyl, $C_1$-$C_2$-Fluoroalkyl oder -chloroalkyl, N,N-Dimethylaminoethyl, Pyrrolidi-

nylethyl, $C_1$-$C_4$-Alkylthio, Phenylthio, Methylphenylthio, Chlorophenylthio, Alkylcarbonyl mit 1 bis 4 Kohlenstoff-Atomen in der Alkyl-Struktureinheit, Phenylmethylthio, Cyano, Alkoxycarbonyl mit 1 bis 4 Kohlenstoff-Atomen in der Alkyl-Struktureinheit oder Phenoxycarbonyl bezeichnet.

3. Verbindungen der Formel (I) nach Anspruch 1, dadurch gekennzeichnet, daß
$R^1$ Trifluoromethyl oder Trifluoromethoxy ist,
X Fluor oder Chlor ist,
Z ein Schwefel-Atom ist,
Y Fluor ist,
n 0 oder 1 ist,
Q ein Sauerstoff-oder Schwefel-Atom ist und
$R^2$ Wasserstoff, Methyl, Ethyl, Phenyl, Acetyl oder Phenylthio ist.

4. Verbindungen nach Ansprüchen 1 bis 3, ausgewählt aus S-Wasserstoff-N-(6-trifluoromethoxybenzothiazol-2-yl)-2,6-difluorobenzothioimidat der Formel

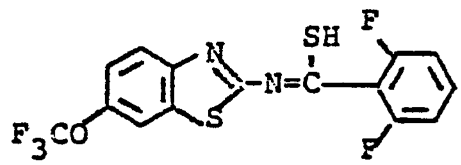

,

Methyl-N-(6-trifluoromethoxybenzothiazol-2-yl)-2,6-difluorobenzimidat der Formel

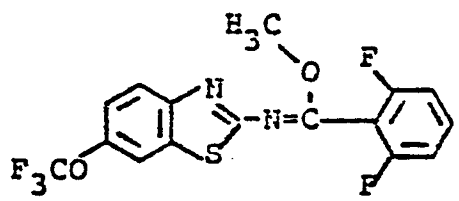

,

Ethyl-N-(6-trifluoromethoxybenzothiazol-2-yl)-2,6-difluorobenzimidat der Formel

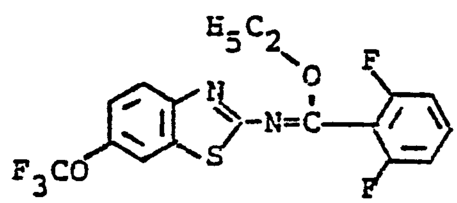

,

Isopropyl-N-(6-trifluoromethoxybenzothiazol-2-yl)-2,6-difluorobenzimidat der Formel

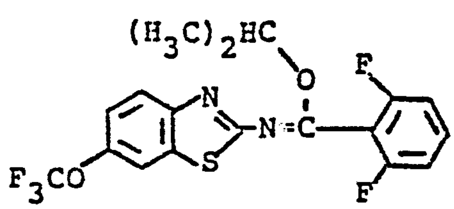

,

Phenyl-N-(6-trifluoromethoxybenzothiazol-2-yl)-2,6-difluorobenzimidat der Formel

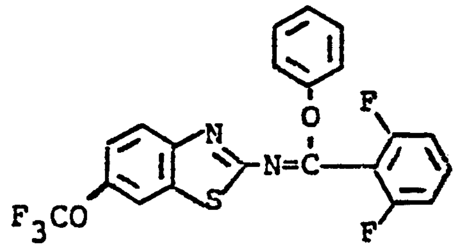

und

S-Acetyl-N-(6-trifluoromethoxybenzothiazol-2-yl)-2,6-difluorobenzothioimidat der Formel

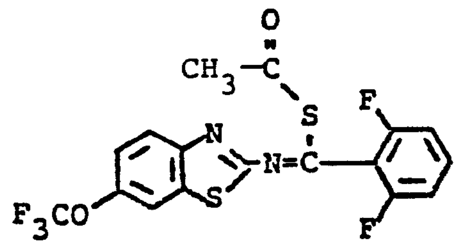

.

5. Verfahren zur Herstellung der Benzimidate der Formel (I)

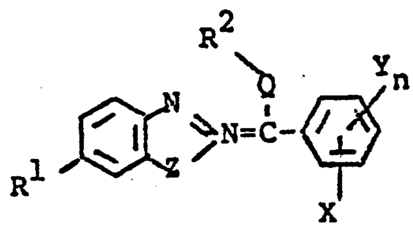

(I)

,

in der

$R^1$ Halogen, Halogenoalkyl, Halogenoalkoxy, Halogenoalkylthio, Halogenoalkylsulfinyl oder Halogenoalkylsulfonyl bezeichnet,

X Halogen oder Alkyl bezeichnet,

Z Sauerstoff oder Schwefel bezeichnet,

Y Halogen oder Alkyl bezeichnet,

n 0, 1 oder 2 ist,

Q Sauerstoff, Schwefel oder die Gruppe $>$N-$R^3$ bezeichnet, in der

$R^3$ Wasserstoff oder Alkyl bezeichnet, und

$R^2$ Wasserstoff, Alkyl, Halogenoalkyl, Alkoxyalkyl, Alkylthioalkyl, N,N-disubstituiertes Aminoalkyl, Aralkyl, Aryloxyalkyl, Arylthioalkyl, Cycloalkyl, Alkenyl, Alkinyl, Aryl, Alkylthio, Aralkylthio, Arylthio, Alkylcarbonyl, Alkoxycarbonyl, Aryloxycarbonyl oder Cyano bezeichnet,

dadurch gekennzeichnet, daß

(a) in dem Fall, in dem $R^2$ in der Formel (I) Wasserstoff, Alkyl, Alkenyl, Alkinyl, Aryl, Cycloalkyl, Alkoxyalkyl, Alkylthioalkyl, Aryloxyalkyl, Arylthioalkyl, Aralkyl, Halogenoalkyl oder N,N-disubstituiertes Aminoalkyl bezeichnet, wobei in diesem Fall in der nachstehenden Formel (III) $R^2$ durch das Symtol $R^4$ ersetzt ist,

Verbindungen der Formel (II)

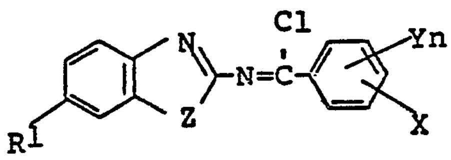

(II)

,

in der

$R^1$, X, Y, Z und n die gleichen Bedeutungen wie oben angegeben haben,

mit den Verbindungen der Formel (III)

$R^4$-Q-H (III),

in der $R^4$ und Q die im Vorstehenden angegebenen Bedeutungen haben, in Gegenwart inerter Lösungsmittel und in Gegenwart einer Base umgesetzt werden oder

(b) in dem Fall, in dem $R^2$ in der Formel (I) Alkyl, Alkenyl, Alkinyl, Cycloalkyl, Alkoxyalkyl, Alkylthioalkyl, Aryloxyalkyl, Arylthioalkyl, Aralkyl, Halogenoalkyl oder N,N-disubstituiertes Aminoalkyl bezeichnet und Q in der Formel (I) Schwefel ist, wobei in diesem Fall in der nachstehenden Formel (V) $R^2$ durch das Symbol $R^5$ ersetzt ist,

Verbindungen der Formel (IV)

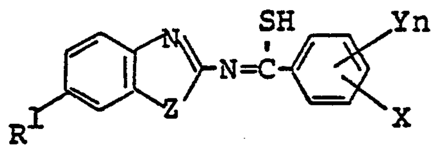

(IV)

,

in der

$R^1$, X, Y, Z und n die gleichen Bedeutungen wie oben angegeben haben,

mit Verbindungen der Formel (V)

$R^5$-M (V),

in der $R^5$ die im Vorstehenden angegebenen Bedeutungen hat und M Brom, Iod oder $-OSO_2-L$ bezeichnet, worin L für Alkyl oder Aryl steht, in Gegenwart inerter Lösungsmittel, gegebenenfalls in Gegenwart einer Base, umgesetzt werden oder

(c) in dem Fall, in dem $R^2$ in der Formel (I) Alkylthio, Arylthio, Alkylcarbonyl, Arylalkylthio, Alkoxycarbonyl oder Aryloxycarbonyl bezeichnet und Q in der Formel (I) Aryloxycarbonyl bezeichnet und Q in der Formel (I) Schwefel ist, wobei in diesem Fall in der nachstehenden Formel (VI) $R^2$ durch $R^6$ ersetzt ist, die vorstehenden Verbindungen der Formel (IV) mit Verbindungen der Formel (VI)

$R^6$-Cl (VI),

in der $R^6$ die im Vorstehenden angegebenen Bedeutungen hat, in Gegenwart inerter Lösungsmittel und in Gegenwart einer Base umgesetzt werden oder

(d) in dem Fall, in dem in der Formel (I) $R^2$ Cyano ist und Q Schwefel ist, die vorstehenden Verbindungen der Formel (I) mit Thiocyanaten in Gegenwart inerter Lösungsmittel umgesetzt werden oder

(e) in dem Fall, in dem in der Formel (I) $R^2$ Cyano ist und Q Schwefel ist, Verbindungen der Formel - (VII)

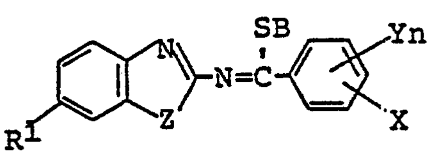

(VII)

,

in der

$R^1$, X, Y, Z und n die gleichen Bedeutungen wie oben angegeben haben und B ein Alkalimetall, ein Äquivalent eines Erdalkalimetalls oder eine Ammonium-Gruppe bezeichnet, mit Bromocyan in Gegenwart inerter Lösungsmittel umgesetzt werden oder

(f) in dem Fall, in dem $R^2$ in der Formel (I) die im Vorstehenden in der Verfahrensvariante (a) für $R^4$ angegebenen Bedeutungen hat und Q für $>N-R^3$ steht, Verbindungen der Formel (Ib)

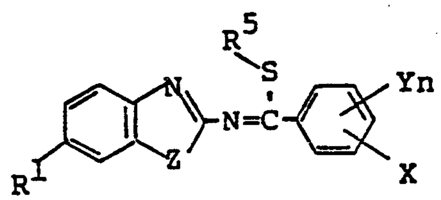 (Ib)

,

in der

$R^1$, X, Y, Z und n die gleichen Bedeutungen wie oben angegeben haben und $R^5$ die im Vorstehenden in der Verfahrensvariante (b) angegebenen Bedeutungen hat, mit Verbindungen der Formel (VIII)

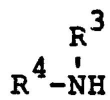 (VIII),

in der

$R^3$ und $R^4$ die im Vorstehenden angegebenen Bedeutungen haben, in Gegenwart inerter Lösungsmittel umgesetzt werden.

6. Pestizide, vorzugsweise insektizide und/oder akarizide, Mittel, dadurch gekennzeichnet, daß sie wenigstens ein Benzimidat der Formel (I) nach den Ansprüchen 1 bis 5 enthalten.

7. Verfahren zur Bekampfung von Schädlingen, insbesondere von Insekten oder Milben, dadurch gekennzeichnet, daß man Benzimidate der Formel (I) nach den Ansprüchen 1 bis 5 auf die Schädlinge und/oder ihren Lebensraum einwirken läßt.

8. Verwendung von Benzimidaten der Formel (I) nach den Ansprüchen 1 bis 5 zur Bekampfung von Schädlingen, insbesondere von Insekten oder Milben

9. Verfahren zur Herstellung von pestiziden, insbesondere insektiziden und/oder akariziden, Mitteln, dadurch gekennzeichnet, daß Benzimidate der Formel (I) nach den Ansprüchen 1 bis 5 mit Streckmitteln und/oder grenzflächenaktiven Mitteln vermischt werden.

Europäisches Patentamt

# EUROPÄISCHER RECHERCHENBERICHT

Nummer der Anmeldung

EP 86115193.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | DE - A1 - 3 419 994 (HOECHST AG)<br>* Ansprüche 1,5 *<br>-- | 1,5,6-9 | C 07 D 277/82<br>C 07 D 263/58<br>A 01 N 43/78<br>A 01 N 43/76 |
| A | GB - A - 1 483 684 (NIPPON SODA COMPANY LTD)<br>* Ansprüche 1,4 *<br>---- | 1,5,6-9 | |
| | | | RECHERCHIERTE SACHGEBIETE (Int. Cl.4) |
| | | | C 07 D 277/00<br>C 07 D 263/00 |

Der vorliegende Recherchenbericht wurde für alle Patentansprüche erstellt.

| Recherchenort | Abschlußdatum der Recherche | Prüfer |
|---|---|---|
| WIEN | 17-02-1987 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument